# EUROPEAN PATENT APPLICATION

(11) **EP 4 618 100 A1**
(43) Date of publication of application: **17.09.2025**
(21) Application number: 25160232.2
(22) Date of filing: 26.02.2025
(51) Int. Cl.: G16H 50/20, G16H 50/70, G16B 40/20

(54) **PREDICTION METHOD AND SYSTEM FOR ACUTE KIDNEY DISEASE**

(30) Priority: 15.03.2024 TW 113109778; 15.03.2024 US 202418606181
(71) Applicant: Taipei Medical University, Taipei City 110 (TW)
(72) Inventor: WU, MAI SZU, 110 Taipei City (TW); LIN, CHIOU FENG, 110 Taipei City (TW); WU, MEI YI, 110 Taipei City (TW); FENG, PO HAO, 110 Taipei City (TW)
(74) Representative: Cabinet Chaillot

(57) **Abstract**

Embodiments of the present disclosure are directed to a prediction method and system for acute kidney disease. The prediction method includes the following steps: inputting a plurality of immune cell population data, a plurality of serum creatinine values, and a plurality of blood urea nitrogen values through an input device, and storing the immune cell population data, the serum creatinine values, and the blood urea nitrogen values in a storage device; accessing a processor to the storage device, and using the immune cell population data, the serum creatinine values, and the blood urea nitrogen values as parameters to establish an acute kidney disease prediction model with a decision tree algorithm; obtaining an immune cell population data, a serum creatinine value, and a blood urea nitrogen value assessed through the input device, and using the processor to perform an interpretation program to obtain an interpretation result of acute kidney disease; outputting the interpretation result of acute kidney disease through an output device. The system includes an input device, a storage device, a processor, and an output device.

## Description

### BACKGROUND OF THE INVENTION

### 1. FIELD OF THE PRESENT DISCLOSURE

The present disclosure relates to a method for predicting acute kidney disease and a system thereof, more particularly, to a method and system for accurately predicting the progression of acute kidney disease by using a decision tree (DT) algorithm to analyze immune cell population data obtained through flow cytometry analysis, combined with serum creatinine (cre) and blood urea nitrogen (BUN) values.

### 2.BACKGROUND

Acute kidney disease (AKD) is a common severe illness in intensive care units (ICUs), which is not immediately diagnosed in real-time during the ICU period, while AKD is diagnosed in patients characterized by continued abnormal renal function following acute kidney injury for seven days. Surveys have shown that the prevalence of AKI in ICUs is as high and half of them may develop AKD. In clinical, AKD patients may need intensive care while it is possible to develop chronic kidney disease, which requires advanced medications, such as blood dialysis and kidney transplantation.

In addition, most patients may experience sepsis and acute kidney injury due to bacterial infections caused by different pathways such as wounds, mucous membranes, or respiratory tract during the treatment process. Furthermore, the causes of acute kidney disease are complex and rapidly changing, making it a significant challenge for medical staff to grasp the timing of treatment, especially whether it will lead to acute kidney disease and require reasonable medical treatment in advance.

On the other hand, the clinical indicators used to evaluate whether acute kidney disease would occur in the past were serum creatinine and blood urea nitrogen. In patients with symptoms of acute kidney disease, their concentrations would abnormally increase, so this can be used to determine whether the patient will experience acute kidney disease. However, due to the different physical conditions of different patients, the accuracy of using serum creatinine and blood urea nitrogen as clinical indicators is still low.

With the rapid development of technology, artificial intelligence is gradually being applied to various medical diagnostic technologies. The use of computer methods to assist in the study of related problems in the medical and biological fields is a powerful tool.

In summary, there is an urgent need for early, influential, and accurate evaluation methods to intervene in the clinical practice of whether acute kidney disease will occur, to provide medical staff with reasonable medical treatment in advance, to reduce the risk of concurrent acute kidney disease, and to improve patient survival rate.

### SUMMARY OF THE INVENTION

Given the evaluation problem of whether acute kidney disease will occur in the knowledge mentioned above, the purpose of the present disclosure is to provide a method and system for predicting acute kidney disease, to reduce the problem of misjudgment caused by human evaluation and the difficulty of quickly selecting medical decisions.

According to one of the purposes of the present disclosure, a method for predicting acute kidney disease is proposed, which comprises the following steps: inputting a plurality of immune cell population data, a plurality of serum creatinine values, and a plurality of blood urea nitrogen values through an input device, and storing the immune cell population data, the serum creatinine values, and the blood urea nitrogen values in a storage device; accessing a processor to the storage device, and using the immune cell population data, the serum creatinine values, and the blood urea nitrogen value as parameters to establish an acute kidney disease prediction model with a decision tree algorithm; obtaining an immune cell population data, a serum creatinine value, and a blood urea nitrogen value assessed through the input device, and using the processor to perform an interpretation program to obtain an interpretation result of acute kidney disease; outputting the interpretation result of acute kidney disease through an output device. Among them, the above steps are all completed by computer software.

The immune cell population data, the serum creatinine values, and the blood urea nitrogen values were obtained by collecting peripheral blood samples from patients who may have concurrent acute kidney disease, and the immune cell population data were obtained by analyzing the peripheral blood samples using a flow cytometer.

The immune cell population data are specifically 55 types of immune cell population data, so it has 55 feature points for machine learning. The 55 types of immune cell population data are shown in Table 1.

**Table 1. 55 types of immune cell population data according to the present disclosure.**

| No. | Cell Subset | Full Name |
|---|---|---|
| 1 | B | B cell |
| 2 | T | T cell |
| 3 | Th | T helper cell |
| 4 | Active Th (HLADR⁺) | Active T helper cell (human leukocyte antigen-DR isotype⁺) |
| 5 | Naïve Th (CD62L⁺) | Naïve T helper cell (CD62L⁺) |
| 6 | Naïve Th (CD45RA⁺) | Naïve T helper cell (CD45RA⁺) |
| 7 | Memory Th (CD62L⁻HLADR⁺) | Memory T helper cell (CD62L⁻ human leukocyte antigen-DR isotype⁺) |
| 8 | Memory Th (CD45RO⁺) | Memory T helper cell (CD45RO⁺) |
| 9 | Regulatory Th | Regulatory T helper cell |
| 10 | Th1 | Type 1 T helper cell |
| 11 | Naïve Th1 | Naïve Type 1 T helper cell |
| 12 | Memory Th1 | Memory Type 1 T helper cell |
| 13 | Th2 | Type 2 T helper cell |
| 14 | Naïve Th2 | Naïve Type 2 T helper cell |
| 15 | Memory Th2 | Memory Type 2 T helper cell |
| 16 | T_{reg} | Regulatory T cell |
| 17 | Naïve T_{reg} | Naïve Regulatory T cell |
| 18 | Memory T_{reg} | Memory Regulatory T cell |
| 19 | Th17 | Type 17 T helper cell |
| 20 | Naïve Th17 | Naïve Type 17 T helper cell |
| 21 | Memory Th17 | Memory Type 17 T helper cell |
| 22 | Th22 | Type 22 T helper cell |
| 23 | Naïve Th22 | Naïve Type 22 T helper cell |
| 24 | Memory Th22 | Memory Type 22 T helper cell |
| 25 | T_{FH} | Follicular helper T cell |
| 26 | Naïve T_{FH} | Naïve Follicular helper T cell |
| 27 | Memory T_{FH} | Memory Follicular helper T cell |
| 28 | T_{C} | Cytotoxic T cell |
| 29 | Active T_{C} | Active Cytotoxic T cell |
| 30 | Naïve T_{C} | Naïve Cytotoxic T cell |
| 31 | Memory T_{C} | Memory Cytotoxic T cell |
| 32 | Regulatory T_{C} | Regulatory Cytotoxic T cell |
| 33 | DP T | Double positive T cell |
| 34 | DN T | Double negative T cell |
| 35 | NK | Natural killer cell |
| 36 | NK CD56b | Natural killer cell CD56b |
| 37 | NK CD56d | Natural killer cell CD56d |
| 38 | CD56b | CD56b cell |
| 39 | CD56d | CD56d cell |
| 40 | NKT | Natural killer T cell |
| 41 | NKT CD8 | Natural killer T cell CD8 |
| 42 | NKT CD4 | Natural killer T cell CD4 |
| 43 | NKT DP | Natural killer T cell Double positive |
| 44 | NKT DN | Natural killer T cell Double negative |
| 45 | CD56b NKT | CD56b Natural killer T cell |
| 46 | CD56d NKT | CD56d Natural killer T cell |
| 47 | CD56b | CD56b CD16⁻ cell |
| 48 | CD56d | CD56d CD16⁻ cell |
| 49 | DC | Dendritic cell |
| 50 | Mature DC | Mature Dendritic cell |
| 51 | Immature DC | Immature Dendritic cell |
| 52 | Monocyte | monocyte |
| 53 | C. Monocyte | classical monocyte |
| 54 | NC. Monocyte | non-classical monocyte |
| 55 | Inter. Monocyte | intermediate monocyte |

Next, use the immune cell population data, the serum creatinine values, and the blood urea nitrogen value as parameters to train the decision tree algorithm. By using supervised learning, these parameters are divided into a training group, a validation group, and a testing group, and the model is continuously trained using a cyclic approach. When the testing group reaches the optimal accuracy, identify the key feature points of the optimal decision tree, which are matched with critical immune cell population data of serum creatinine value and blood urea nitrogen value, to obtain the acute kidney disease prediction model.

Finally, the immune cell population data, the serum creatinine values, and the blood urea nitrogen values are obtained, and the interpretation program based on the acute kidney disease prediction model to obtain the interpretation result of acute kidney disease is performed. Based on the interpretation result of acute kidney disease, it can be determined whether the patient to be evaluated will develop acute kidney disease, in order to facilitate medical staff to make reasonable medical arrangements in advance. According to another purpose of the present disclosure, an acute kidney disease prediction system is proposed, which comprises an input device, a storage device, a processor, and an output device. Among them, the input device is used to input a plurality of immune cell population data, a plurality of serum creatinine values, and a plurality of blood urea nitrogen values, and an immune cell population data, a serum creatinine value, and a blood urea nitrogen value to be evaluated. The storage device is connected to the input device to store the immune cell population data, the serum creatinine values, and the blood urea nitrogen values, and the immune cell population data, the serum creatinine value, and the blood urea nitrogen value to be evaluated. The output device is connected to the storage device to output the interpretation result of acute kidney disease. The processor is connected to a storage device and executes a plurality of instructions to perform the following steps: using the immune cell population data, the serum creatinine values, and the blood urea nitrogen values as parameters to establish an acute kidney disease prediction model with a decision tree algorithm; obtaining an immune cell population data, a serum creatinine value, and a blood urea nitrogen value and performing an interpretation program based on the acute kidney disease prediction model to obtain an interpretation result of acute kidney disease; and outputting the interpretation result of acute kidney disease by accessing the storage device through the output device.

As mentioned above, using the acute kidney disease prediction method and system of the present disclosure, it is possible, at the earliest possible time, to quickly and accurately determine whether a patient will develop acute kidney disease, reduce the burden on nursing staff and physicians, and reduce the problem of inconsistent judgment standards among different nursing staff and physicians. This facilitates subsequent nursing staff and physicians to evaluate whether acute kidney disease will occur more accurately and to make corresponding medical decisions as soon as possible.

### BRIEF DESCRIPTION OF THE DRAWINGS

To describe the technical solutions in the embodiments of this application more clearly, the following briefly introduces the accompanying drawings required for describing the embodiments. Apparently, the accompanying drawings in the following description show merely some embodiments of this application, and a person of ordinary skill in the art may still derive other drawings from these accompanying drawings without creative efforts.
Fig. 1 is a flowchart of an acute kidney disease prediction method according to one embodiment of the present disclosure.
Fig. 2A is a schematic diagram of a decision tree structure obtained by using a plurality of serum creatinine values combined with a plurality of immune cell population data as parameters according to one embodiment of the present disclosure.
Fig. 2B shows an analysis of the accuracy of an acute kidney disease prediction model established using the decision tree structure of Fig. 2A.
Fig. 3A is a schematic diagram of the decision tree structure obtained by using a plurality of blood urea nitrogen values combined with the immune cell population data as parameters according to one embodiment of the present disclosure.
Fig. 3B shows the analysis of the accuracy of the acute kidney disease prediction model established using the decision tree structure of Fig. 3A.
Fig. 4A is a schematic diagram of the decision tree structure obtained using a serum creatinine value and a blood urea nitrogen value as parameters according to one embodiment of the present disclosure.
Fig. 4B shows the analysis of the accuracy of the acute kidney disease prediction model established using the decision tree structure of Fig. 4A.
Fig. 5A is a schematic diagram of the decision tree structure obtained using the serum creatinine value and the blood urea nitrogen value further combined with Naïve Regulatory T cell and Natural killer cell CD56d as parameters according to one embodiment of the present disclosure.
Fig. 5B shows the analysis of the accuracy of the acute kidney disease prediction model established using the decision tree structure of Figure 5A.
Fig. 6 is a schematic diagram of an acute kidney disease prediction system according to one embodiment of the present disclosure.

### DESCRIPTION OF THE EMBODIMENTS

In order to facilitate the review committee's understanding of the technical features, content, advantages, and achievable effects of the present disclosure, the present disclosure is now accompanied by the accompanying drawings and explained in detail in the form of embodiments. The primary purpose of the drawings used is only for illustrative and auxiliary purposes and may not necessarily be the proper proportion and precise configuration after the implementation of the present disclosure. Therefore, the proportion and configuration relationship of the attached drawings should not be interpreted. The scope of rights limited to the actual implementation of the present disclosure shall be described in advance.

Unless otherwise defined, all terms used in this article (including technical and scientific terms) have the meanings commonly understood by those knowledgeable in the technical field to which the present disclosure belongs. It will be further understood that terms such as those defined in commonly used dictionaries should be interpreted as having meanings consistent with their meanings in the context of the relevant technology and the present disclosure and will not be interpreted as idealized or overly formal unless explicitly defined in this context.

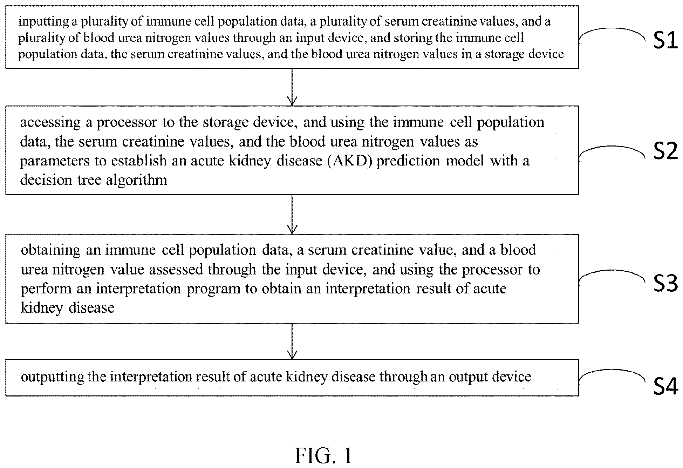

Please refer to Fig. 1, which is a flowchart of an acute kidney disease prediction method according to one embodiment of the present disclosure. As shown in Fig. 1, the prediction method for acute kidney disease comprises the following steps (S1~S4):
S1: inputting a plurality of immune cell population data, a plurality of serum creatinine values, and a plurality of blood urea nitrogen values through an input device and storing the immune cell population data, the serum creatinine values, and the blood urea nitrogen values in a storage device.

The immune cell population data, the serum creatinine values, and the blood urea nitrogen values that can be collected are input into the storage device of the system through the input device. The input device mentioned here is not limited to flow cytometers that can obtain immune cell population data, as well as other analyzers that can obtain the serum creatinine values and the blood urea nitrogen values. The immune cell population data, the serum creatinine values, and the blood urea nitrogen values stored in the database of medical institutions can also be transmitted through physical circuits, storage devices, or wired or wireless networks to input the immune cell population data, serum creatinine values, and the blood urea nitrogen values into the registry as training data for model construction.

Among them, the immune cell population data were obtained by collecting peripheral blood samples from patients who may have concurrent acute kidney disease, and analyzing the peripheral blood samples using a flow cytometer. Therefore, the peripheral blood samples of every patient who may have concurrent acute kidney disease have the immune cell population data, precisely 55 immune cell population data, which can be described in Table 1 above.

S2: accessing a processor to the storage device, and using the immune cell population data, the serum creatinine values, and the blood urea nitrogen values as parameters to establish an acute kidney disease prediction model with a decision tree algorithm.

The processor reads the immune cell population data, the serum creatinine values, and the blood urea nitrogen values stored in the storage device. It uses immune cell population data (i.e., those above 55 immune cell population data) as well as serum creatinine values, blood urea nitrogen values, and other numerical values as training parameters. A decision tree algorithm is used for training, and a model is continuously trained using a cyclic approach. When a testing group reaches the optimal accuracy, identify the key feature points of the optimal decision tree (i.e., critical immune cell population data) to obtain an acute kidney disease prediction model.

Among them, the above parameters obtained from patients who may have concurrent acute kidney disease are divided into a training group and a testing group based on the total number of patients to establish an acute kidney disease prediction model. The ratio of the training group to the testing group is 8:2, which means 80% of the training group and 20% of the testing group. However, the actual number of patients is 72, so the training group has 57 patients, while the testing group has 15 patients.

Afterward, the trained acute kidney disease prediction model was used to select 106 different patients who may have concurrent acute kidney disease as the training group. The acute kidney disease prediction model was retrained again, and 32 additional patients who may have concurrent acute kidney disease were selected as a validation group to confirm the prediction accuracy of the retrained acute kidney disease prediction model. The personal medical information of 106 patients in the training group and 32 patients in the validation group are shown in Table 2. The 106 patients in the training group were identified as patients from 2020 to 2021, while the 32 patients in the validation group were identified as patients from 2022. By increasing the number of samples in the training group, the prediction accuracy of the trained acute kidney disease prediction model can be further improved.

**Table 2. Personal medical information according to the present disclosure.**

| statistical data | training group (2020~2021) Sepsis-associated acute kidney disease (SA-AKD) | | | validation group (2022) Sepsis-associated acute kidney disease (SA-AKD) | | |
|---|---|---|---|---|---|---|
| | negative (Sample size=70) | positive (Sample size=36) | P value | negative (Sample size=27) | positive (Sample size=5) | P value |
| Age (years old) | 66 ± 18 | 67 ± 19 | 0.826 | 65 ± 17 | 82 ± 11 | 0.030 |

| Gender | | | | | | |
|---|---|---|---|---|---|---|
| number of female (n) | 34 | 11 | 0.098 | 17 | 3 | 1.000 |
| number of male (n) | 36 | 25 | | 10 | 2 | |
| number of inpatients (n) | 65 | 35 | 0.661 | 21 | 4 | 1.000 |
| Hospitalization days (days) | 5 ± 4 | 7 ± 5 | 0.589 | 4 ± 3 | 4 ± 2 | 0.584 |
| Rapid sequential organ failure score (qSOFA) | 11 | 6 | 1.000 | 6 | 1 | 1.000 |

| Bacterial species | | | | | | |
|---|---|---|---|---|---|---|
| Gram-positive bacteria (GPC) (n) | 20 | 15 | 0.177 | 3 | 2 | 0.216 |
| Gram-negative bacteria (GNB) (n) | 47 | 18 | | 21 | 3 | |
| GPC+GNB (n) | 2 | 3 | | 3 | 0 | |
| number of fungi (n) | 1 | 0 | | 0 | 0 | |

| Clinical indicators | | | | | | |
|---|---|---|---|---|---|---|
| Blood urea nitrogen (mg/dL) | 15.96 ± 14.54 | 29.59 ± 21.19 | ≤ 0.0001 | 16.41 ± 13.17 | 47.60 ± 36.90 | 0.003 |
| Serum creatinine (mg/dL) | 0.957 ± 0.630 | 2.700 ± 6.175 | ≤ 0.0001 | 0.879 ± 0.456 | 1.840 ± 0.826 | 0.001 |

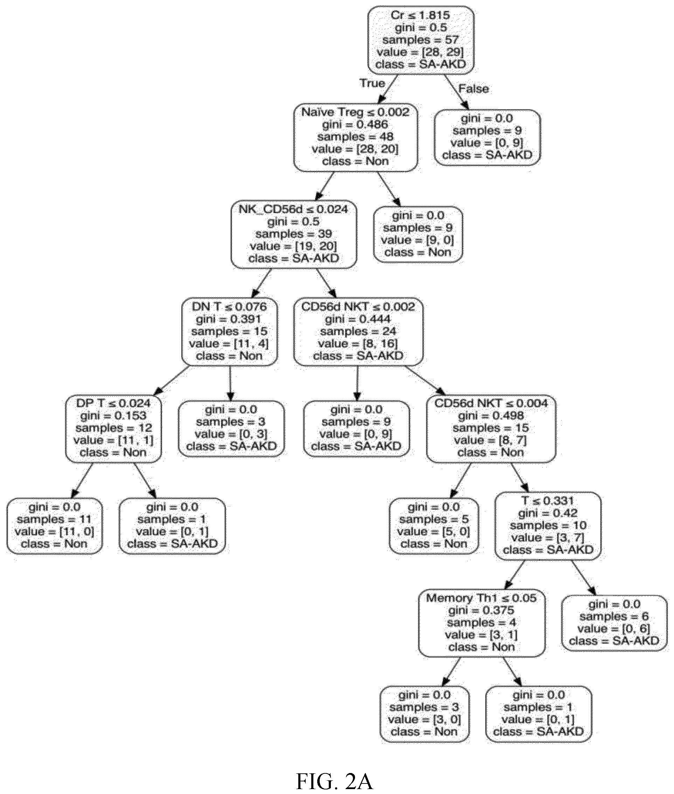

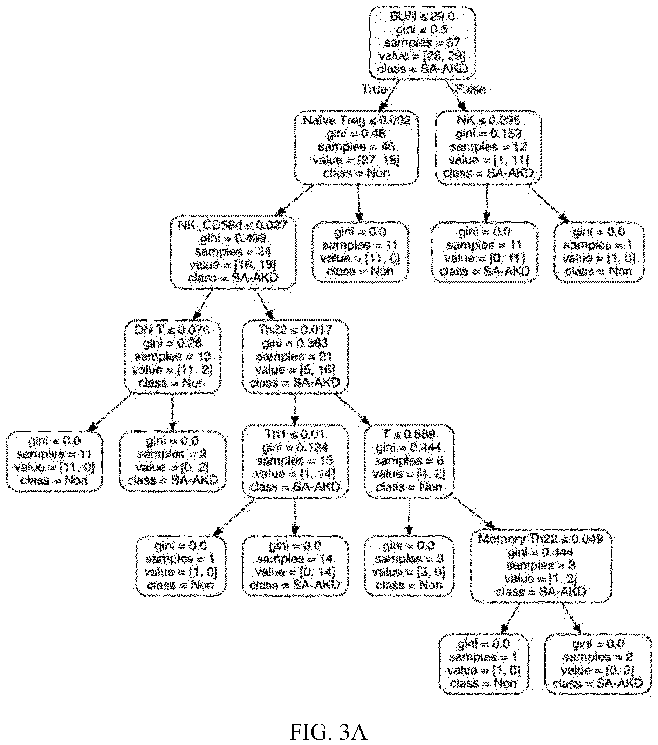

Please refer to Figs. 2A and 3A together, which are schematic diagrams of the decision tree structure obtained by using the serum creatinine values combined with the immune cell population data as parameters in the embodiments of the present disclosure, as well as schematic diagrams of the decision tree structure obtained by using the blood urea nitrogen values combined with the immune cell population data as parameters.

In the decision tree structure diagram, Cr represents serum creatinine value, BUN represents blood urea nitrogen value, and gini is the Gini coefficient, which is used to represent the average equality of the classified groups (generally speaking, when the gini is 0, it represents complete equality, below 0.2 represents high equality, 0.2~0.3 represents still equal, 0.3~0.4 represents tolerable, 0.4~0.6 represents significant differences, above 0.6 represents high inequality, and 1.0 represents complete inequality), while data represent the total number of people, and value is the number of people classified by machine learning whether acute kidney disease will occur. Class is a classification group. If it is sepsis-associated acute kidney disease (SA-AKD), it indicates that it is classified as acute kidney disease related to sepsis. If it is not SA-AKD, it is represented as Non.

For example, in the first level of the decision tree structure in Fig. 2A, the serum creatinine value ≤1.185 is the threshold for classification, with a total of 57 people and a value of [28,29]. The value on the left side, 28, represents non-SA-AKD, while the value on the right side, 29, represents SA-AKD. The ratio of the two is converted to a gini of about 0.5. Due to the large number of SA-AKD people, this level is classified as SA-AKD. Next, in the second level, the threshold for classification is based on immune cell Naïve Regulatory T cell (Naïve T_{reg}) ≤ 0.002. Among 57 individuals, 9 individuals have Naïve T_{reg} >0.002, with a value of [0,9] and a gini of 0.0, indicating complete equality. These 9 individuals are all SA-AKD patients, so the further classification is stopped, while the other 48 individuals have a value of [28,20] and a gini of 0.486. Moreover, there are more non-SA-AKD individuals who are classified as Non and continue to be further classified. By analogy, continue to classify different immune cells based on their threshold values until the gini reaches 0.0 in order to complete the decision tree structure.

Furthermore, the critical feature points of the optimal decision tree are obtained by selecting the classification nodes of the decision tree as weights. The higher the weight of the immune cells used by the upper level classification nodes, the higher their contribution and importance in classifying whether acute kidney disease related to sepsis will occur.

From Figs. 2A and 3A, it can be seen that the decision tree structure constructed by combining the serum creatinine value and the blood urea nitrogen value with the immune cell population data has Naïve T_{reg} in the second level and NK CD56d in the third level, indicating that the weight of these two immune cells is relatively high. Therefore, the critical immune cell population data for the optimal decision tree will be based on these two immune cells, and the serum creatinine values and the blood urea nitrogen values will be used as training parameters to establish an accurate acute kidney disease prediction model.

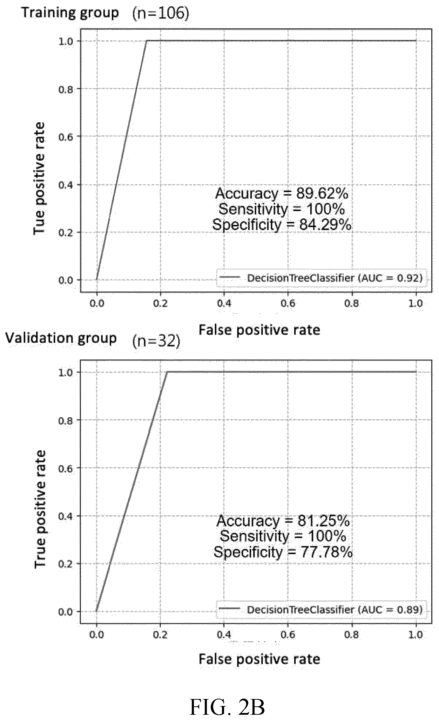

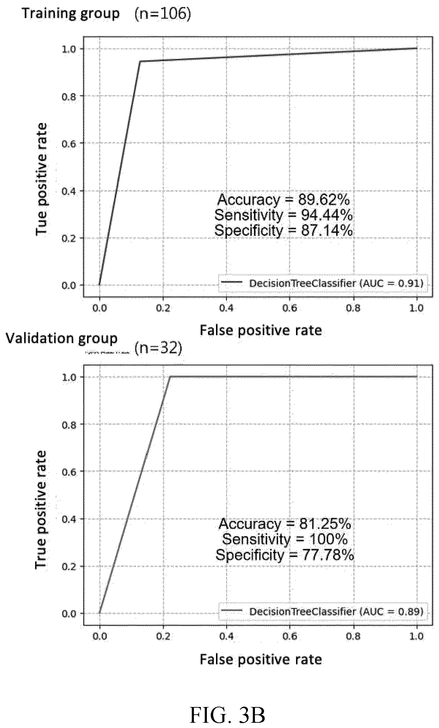

Next, please refer to Figs. 2B and 3B, which are the analysis charts of the accuracy of the acute kidney disease prediction model established using the decision tree framework of Figs. 2A and 3A in the embodiments of the present disclosure. Among them, as mentioned earlier, these two figures were trained with 106 additional patients as the training group, and 32 additional patients were selected as the validation group to confirm the accuracy of the retrained acute kidney disease prediction model. From Figs. 2B and 3B, it can be seen that the prediction accuracy (accuracy) of the training group (106 patients) was 89.62%, sensitivity was 100% and 94.44% in order, and specificity was 84.29% and 87.14% in order. The prediction accuracy, sensitivity, and specificity of the validation group (32 patients) were all 81.25%, 100%, and 77.78%, respectively. From this, it can be seen that the decision tree structure constructed by combining the serum creatinine values or the blood urea nitrogen values with the immune cell population data has a prediction accuracy of over 80%.

On the other hand, in order to confirm the predictive accuracy of the acute kidney disease prediction model constructed by combining the serum creatinine values or the blood urea nitrogen values with the immune cell population data, the predictive accuracy of the serum creatinine values and the blood urea nitrogen values, which are only used as clinical indicators of acute kidney disease, is higher than that of using only conventional knowledge. Therefore, only the serum creatinine value and the blood urea nitrogen value are used as parameters, and a decision tree algorithm is also used to establish an acute kidney disease prediction model for comparison of predictive accuracy.

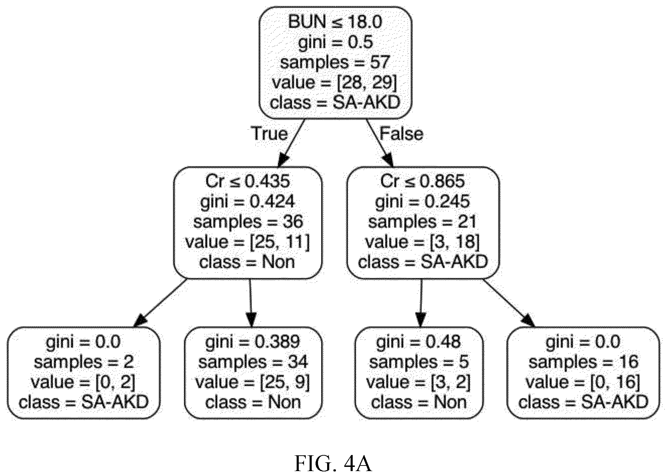

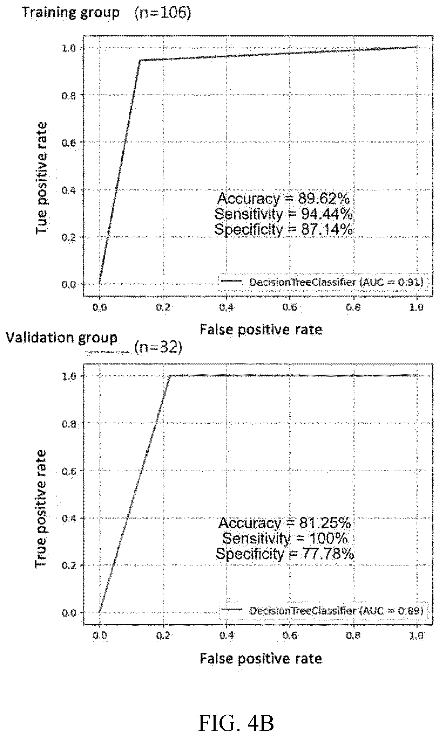

Please refer to Figs. 4A and 4B, which are schematic diagrams of the decision tree structure obtained by using the serum creatinine values and the blood urea nitrogen values as parameters in the embodiments of the present disclosure, as well as the analysis of the prediction accuracy of the acute kidney disease prediction model established using them.

From the decision tree structure in Fig. 4A, it can be seen that using the serum creatinine values and the blood urea nitrogen values can indeed quickly classify SA-AKD patients and non-SA-AKD patients. However, further from Fig. 4B, it can be seen that the acute kidney disease prediction model obtained using only a serum creatinine value and a blood urea nitrogen value as training parameters has a prediction accuracy of 77.36%, sensitivity of 66.67%, and specificity of 82.86% in the training group (106 patients), while the prediction accuracy of 75%, sensitivity of 100%, and specificity of 70.37% in the validation group (32 patients). From this, it can be seen that the prediction accuracy of the acute kidney disease model constructed solely using the serum creatinine value and the blood urea nitrogen value as training parameters is not up to 80%, which is significantly lower than its prediction accuracy when combined with the immune cell population data.

Furthermore, using the immune cell population data, the serum creatinine values, and the blood urea nitrogen values as parameters, a decision tree algorithm was used to establish an acute kidney disease prediction model. It was confirmed whether combining these three parameters can further improve the prediction accuracy.

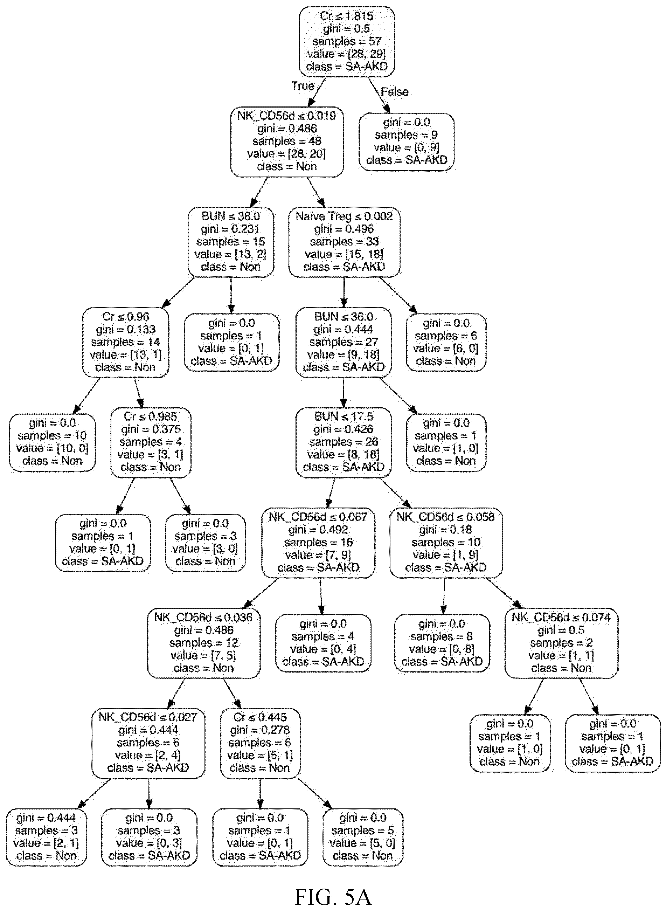

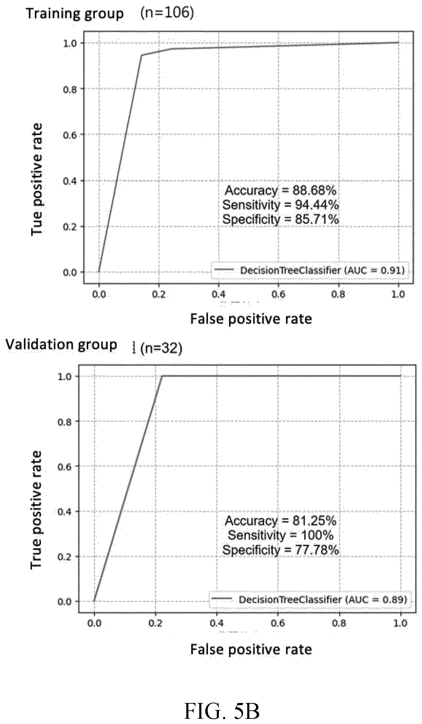

Please refer to Figs. 5A and 5B, which are schematic diagrams of the decision tree structure obtained by using the serum creatinine value and the blood urea nitrogen value, further combined with Naïve T_{reg} and NK CD56d immune cells as parameters in the embodiments of the present disclosure, and the analysis of the prediction accuracy of the acute kidney disease prediction model established using them.

As shown in Figs. 2A and 3A above, it can be seen that Naïve T_{reg} and NK CD56d have a higher proportion of immune cells. Therefore, the combination of these two immune cells with the serum creatinine values and the blood urea nitrogen values was used as training parameters to establish an acute kidney disease prediction model. From Fig. 5B, it can be seen that the acute kidney disease prediction model established by using serum creatinine value and blood urea nitrogen value, combined with Naïve T_{reg} and NK CD56d immune cells as parameters, has a prediction accuracy of 88.68%, the sensitivity of 94.44%, and specificity of 85.71% in the training group (106 patients), while the prediction accuracy of 81.25% and sensitivity of 100% in the validation group (32 patients), and the specificity is 77.78%. From this, it can be seen that using only the two immune cell data with higher classification weights, combined with the serum creatinine value and the blood urea nitrogen value, can achieve a prediction accuracy of over 80%. There is no need to simultaneously obtain those above 55 immune cell population data as parameters to establish an acute kidney disease prediction model. This not only improves the speed of establishing the prediction model but also further improves the efficiency of analyzing peripheral blood samples, obtain immune cell data for both Naïve T_{reg} and NK CD56d.

S3: obtaining immune cell population data, a serum creatinine value, and a blood urea nitrogen value assessed through the input device, and using the processor to perform an interpretation program to obtain an interpretation result of acute kidney disease.

The acute kidney disease prediction model established through the above step S2 is used to evaluate the immune cell population data, the serum creatinine values, and the blood urea nitrogen values to obtain the acute kidney disease interpretation results. Based on the results of acute kidney disease assessment, it can be determined whether the patients to be evaluated will develop acute kidney disease, to facilitate medical staff to make reasonable medical arrangements in advance. The input device described here is the same as mentioned earlier, and will not be further elaborated here.

In addition, to confirm the accuracy of different algorithms in predicting acute kidney disease, the immune cell population data (i.e., those above 55 immune cell population data), the serum creatinine values, and the blood urea nitrogen values were used as parameters. Peripheral blood samples from 106 patients were also analyzed to obtain these parameters, and other machine learning algorithms such as Support Vector Machine (SVM) and K-Nearest Neighbor Algorithm (KNN) were used to establish an acute kidney disease prediction model, which was compared with the acute kidney disease prediction model established by Decision Tree (DT) algorithm. The prediction accuracy of the three algorithms is shown in Table 3.

**Table 3. Prediction accuracy of the algorithms according to the present disclosure.**

| machine learning algorithms | SVM | KNN | DT |
|---|---|---|---|
| prediction accuracy | 70% | 50% | 85% |

According to Table 3, the prediction accuracy of SVM is 70%, while the prediction accuracy of KNN is only 50%, which is far lower than the prediction accuracy of the decision tree algorithm of 85%. From this, it can be seen that the acute kidney disease prediction model established using the decision tree algorithm is superior to other types of algorithms.

S4: Output the interpretation result of acute kidney disease through an output device.

The acute kidney disease diagnosis results obtained through the above step S3 can be further output through the output device. The output device disclosed in this embodiment may include various display interfaces, such as computer screens, displays, or handheld device displays.

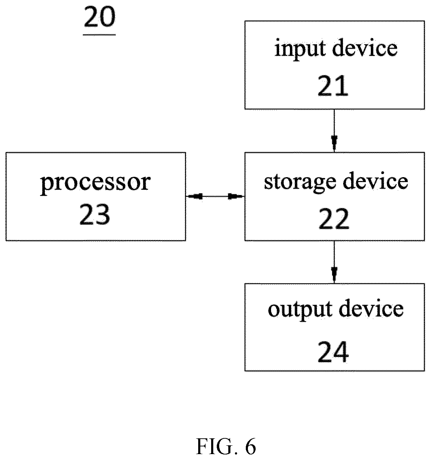

Please refer to Fig. 6, which is a schematic diagram of the acute kidney disease prediction system in one embodiment of the present disclosure. As shown in Fig. 6, the acute kidney disease prediction system 20 may comprise an input device 21, a storage device 22, a processor 23, and an output device 24.

In this embodiment, the input device 21 can be composed of a flow cytometer paired with an analytical device for detecting serum creatinine and blood urea nitrogen. It analyzes peripheral blood samples of patients who may have concurrent acute kidney disease to obtain the immune cell population data, the serum creatinine values, and the blood urea nitrogen values. In another embodiment, the input device 21 is not limited to a flow cytometer and its accompanying analytical device for detecting serum creatinine and blood urea nitrogen. The input device 21 may comprise an input interface for electronic devices such as personal computers, smartphones, servers, etc., including touch screens, keyboards, mice, etc., transmitting the immune cell population data, the serum creatinine values, and the blood urea nitrogen values through file mode. Alternatively, historical data can be transmitted through wireless networks, wireless communication, or general wired internet networks to the memory storage device 22, which may include read-only memory, flash memory, disks, or cloud databases.

Subsequently, the acute kidney disease prediction system 20 accesses the storage device 22 through a processor 23, which may comprise a central processing unit, image processing unit, microprocessor, etc., in a computer or server. It may comprise a multi-core processing unit or a combination of multiple processing units. The processor 23 executes instructions to access the immune cell population data, the serum creatinine values, and the blood urea nitrogen values stored in storage device 22 for training programs and simultaneously takes the immune cell population data, the serum creatinine values, and the blood urea nitrogen values to be evaluated for interpretation programs. Specifically, the training program takes the immune cell population data, the serum creatinine value, and the blood urea nitrogen value from the original storage device 22 as parameters and uses a decision tree algorithm for operation to establish an acute kidney disease prediction model.

Subsequently, the immune cell population data, the serum creatinine values, and the blood urea nitrogen values to be evaluated were analyzed using a judgment program and calculated using the established acute kidney disease prediction model to obtain the results of acute kidney disease judgment. The output device 24 accesses the storage device 22 and outputs the results of acute kidney disease diagnosis. The output device 24 may comprise various display interfaces, such as computer screens, displays, or handheld device displays.

Through the acute kidney disease prediction method and system of the present disclosure, the workload of medical staff and physicians can be significantly reduced, and errors in manual interpretation can be reduced, leading to diagnostic biases in the diagnosis of acute kidney disease. It is also possible to quickly and accurately determine whether patients will develop acute kidney disease. In addition to reducing the burden on nursing staff and physicians and reducing the problem of inconsistent judgment standards among different nursing staff and physicians, it also assists subsequent nursing staff and physicians in accurately evaluating whether acute kidney disease will occur and can make corresponding medical decisions as soon as possible.

The above is only for illustrative purposes and not for restrictive purposes. Any equivalent modifications or changes made to the present disclosure that do not deviate from the spirit and scope of the present disclosure shall be included in the scope of the attached patent application.

## Claims

1. A method for predicting acute kidney disease, comprising:
S1: inputting a plurality of immune cell population data, a plurality of serum creatinine values, and a plurality of blood urea nitrogen values through an input device and storing the immune cell population data, the serum creatinine values, and the blood urea nitrogen values in a storage device;
S2: accessing a processor to the storage device and using the immune cell population data, the serum creatinine values, and the blood urea nitrogen values as parameters to establish an acute kidney disease prediction model with a decision tree algorithm;
S3: obtaining immune cell population data, a serum creatinine value, and a blood urea nitrogen value assessed through the input device, and using the processor to perform an interpretation program to obtain an interpretation result of acute kidney disease; and
S4: outputting the interpretation result of acute kidney disease through an output device;
wherein, the above steps are all completed by computer software.

2. The method for predicting acute kidney disease as claimed in Claim 1, wherein the input device is composed of a first-class cell analyzer paired with an analytical device for detecting serum creatinine and blood urea nitrogen.

3. The method for predicting acute kidney disease as claimed in Claim 1, wherein the immune cell population data are selected from the following 55 immune cell population data: B cell, T cell, T helper cell, Active T helper cell (human leukocyte antigen-DR isotype⁺), Naïve T helper cell (CD62L⁺), Naïve T helper cell (CD45RA⁺), Memory T helper cell (CD62L⁻HLADR⁺), Memory T helper cell (CD45RO+), Regulatory T helper cell, Type 1 T helper cell, Naïve Type 1 T helper cell, Memory Type 1 T helper cell, Type 2 T helper cell, Naïve Type 2 T helper cell, Memory Type 2 T helper cell, Regulatory T cell, Naïve Regulatory T cell, Memory Regulatory T cell, Type 17 T helper cell, Naïve Type 17 T helper cell, Memory Type 17 T helper cell, Type 22 T helper cell, Naïve Type 22 T helper cell, Memory Type 22 T helper cell, Follicular helper T cell, Naïve Follicular helper T cell, Memory Follicular helper T cell, Cytotoxic T cell, Active Cytotoxic T cell, Naïve Cytotoxic T cell, Memory Cytotoxic T cell, Regulatory Cytotoxic T cell, Double positive T cell, Double negative T cell, Natural killer cell, Natural killer cell CD56b, Natural killer cell CD56d, CD56b cell, CD56d cell, Natural killer T cell, Natural killer T cell CD8, Natural killer T cell CD4, Natural killer T cell Double positive, Natural killer T cell Double negative, CD56b Natural killer T cell, CD56d Natural killer T cell, CD56b CD16⁻ cell, CD56d CD16⁻ cell, Dendritic cell, Mature Dendritic cell, Immature Dendritic cell, monocyte, classical monocyte, non-classical monocyte, intermediate monocyte.

4. The method for predicting acute kidney disease as claimed in any one of Claims 1 to 3, wherein the acute kidney disease prediction model comprises an optimal decision tree with two critical immune cell populations.

5. The method for predicting acute kidney disease as claimed in Claim 4, wherein the two critical immune cell populations are Naïve Regulatory T cell (Naïve T_{reg}) and Natural killer cell CD56d (NK CD56d), respectively.

6. An acute kidney disease prediction system, comprising:
an input device used to input a plurality of immune cell population data, a plurality of serum creatinine values, and a plurality of blood urea nitrogen values, and an immune cell population data, a serum creatinine value, and a blood urea nitrogen value to be evaluated;
a storage device connected to the input device for storing the immune cell population data, the serum creatinine values, and the blood urea nitrogen values, and the immune cell population data, the serum creatinine value, and the blood urea nitrogen value to be evaluated;
an output device, connected to the storage device for outputting an interpretation results of acute kidney disease; and
a processor connected to the storage device executes a plurality of instructions to perform the following steps:
using the immune cell population data, the serum creatinine values, and the blood urea nitrogen values as parameters to establish an acute kidney disease prediction model with a decision tree algorithm
obtaining an immune cell population data, a serum creatinine value, and a blood urea nitrogen value and performing an interpretation program based on the acute kidney disease prediction model to obtain an interpretation result of acute kidney disease; and
outputting the interpretation result of acute kidney disease by accessing the storage device through the output device.

7. The acute kidney disease prediction system, as claimed in Claim 6, wherein the input device is composed of a first-class cell analyzer paired with an analytical device for detecting serum creatinine and blood urea nitrogen.
